# EUROPÄISCHE PATENTANMELDUNG

(11) **EP 1 806 112 A1**
(43) Veröffentlichungstag der Anmeldung: **11.07.2007**
(21) Anmeldenummer: 06026668.1
(22) Anmeldetag: 22.12.2006
(51) Int. Cl.: A61F 2/34

(54) **Implantat zur Bildung einer Hüftgelenkpfanne**

(30) Priorität: 10.01.2006 DE 102006001430
(71) Anmelder: Siebel, Thomas, 66130 Saarbrücken (DE)
(72) Erfinder: Siebel, Thomas, 66130 Saarbrücken (DE)
(74) Vertreter: Bernhardt, Reinold

(57) **Zusammenfassung**

Die Erfindung betrifft ein Implantat zur Bildung einer künstlichen Hüftgelenkpfanne, mit einer konkaven Außenfläche (1), die zur Anlage gegen in der natürlichen Hüftgelenkpfanne verbliebenes Knorpelgewebe vorgesehen ist. Gemäß der Erfindung weist die Außenfläche (1) wenigstens eine Knorpelgewebe aufnehmende Vertiefung (4-6) oder/und wenigstens einen in das Knorpelgewebe eindringenden Vorsprung auf. Vorzugsweise ist der an die Vertiefung (4-6) oder/und den Vorsprung angrenzende übrige Teil (9-15) der Außenfläche (1) poliert.

## Beschreibung

Die Erfindung betrifft ein Implantat zur Bildung einer künstlichen Hüftgelenkpfanne, mit einer konvexen Außenfläche, die zur Anlage gegen in der natürlichen Hüftgelenkpfanne verbliebenes Knorpelgewebe vorgesehen ist.

Pfannenimplantate, die zum Einsetzen in eine natürliche Hüftgelenkpfanne mit erhalten gebliebender Knorpelbelagschicht vorgesehen sind, gehen aus der DE 103 38 420.0 hervor. Sie kommen zur Anwendung, wenn nur der Femurkopf angegriffen ist und ganz oder teilweise durch eine Prothese ersetzt werden muss, welche dann gegen die Innenfläche des Pfannenimplantats anliegt.
Die Außenfläche der bekannten Pfannenimplantate ist geglättet, so dass das Implantat gegen das Knorpelgewebe gleitbeweglich anliegt. Ein Flansch am Rand des Pfannenimplantats verhindert eine zu weite Verdrehung und damit einen Austritt des Pfannenimplantats aus der Hüftgelenkpfanne.

Der Erfindung liegt die Aufgabe zugrunde, ein neues Pfannenimplantat der eingangs erwähnten Art zu schaffen, das bei geringer Belastung des in der natürlichen Hüftgelenkpfanne verbliebenen Knorpelgewebes ein schnelles Anwachsen des Knorpelgewebes an das Pfannenimplantat ermöglicht.

Das diese Aufgabe lösende Implantat nach der Erfindung ist dadurch gekennzeichnet, dass die Außenfläche wenigstens eine Knorpelgewebe aufnehmende Vertiefung oder/und wenigstens einen in das Knorpelgewebe eindringenden Vorsprung aufweist.

Während der an die Vertiefung bzw. den Vorsprung angrenzende übrige Teil der Außenfläche weiterhin geglättet sein und daher gleitbeweglich gegen das Knorpelgewebe anliegen kann, sorgt die Vertiefung bzw. der Vorsprung für eine gewissen Arretierung des Pfannenimplantats in der natürlichen Hüftgelenkpfanne. Vorteilhaft wird das Implantat in der natürlichen Hüftgelenkpfanne gehalten und das Knorpelgewebe wächst schnell an.

In einer bevorzugten Ausführungsform der Erfindung ist der geglättete Teil der Außenfläche größer als der von der Vertiefung oder/und dem Vorsprung eingenommene Teil. Besondere Beanspruchungen des Knorpelgewebes beschränken sich auf den Bereich der Vertiefung bzw. des Vorsprungs.

Die Vertiefung kann kreisförmig oder in anderer Weise begrenzt sein. Vorzugsweise ist sie länglich und es handelt sich um eine Rille. Bei dem Vorsprung kann es sich z.B. um eine runde Noppe handeln. Vorzugsweise ist der Vorsprung jedoch länglich und insbesondere als Steg ausgebildet.

Die Vertiefung bildet zweckmäßig einen geschlossenen oder unterbrochenen Ring, welcher vorzugsweise parallel zum Rand der Außenfläche verläuft. Ein solcher Ring kann Verdrehungen des Pfannenimplantats innerhalb der Hüftgelenkpfanne in zwei Drehrichtungen entgegenwirken.

Vorzugsweise verläuft der Vorsprung, in Projektion auf eine zur Rotationssymmetrieachse des Implantats senkrechte Projektionsfläche, radial zur Rotationssymmetrieachse. Ein Implantat mit mehreren solchen, stegförmigen Vorsprüngen lässt sich leicht in Richtung der Rotationssymmetrieachse in die natürliche Gelenkpfanne hinein eindrücken. Anders verlaufende Vorsprünge würden das Eindrücken in die Gelenkpfanne behindern.

Der Vorsprung kann sich durchgehend oder mit wenigstens einer Unterbrechung von einem Punkt am Rand des Implantats bis zu einem diesem Punkt diametral gegenüberliegenden Randpunkt erstrecken.

Auf der Außenfläche können sich kreuzende Rillen oder/und Stege gebildet sein.

Es ist denkbar, auf einer Außenfläche sowohl Vertiefungen als auch Vorsprünge vorzusehen.

In einer weiteren Ausführungsform der Erfindung weist die Außenfläche eine Aufrauhung auf, die sich vorzugsweise über die gesamte Außenfläche erstreckt und z.B. in der Art einer Rändelung gebildet sein kann. Zweckmäßig ist die Aufrauhung so stark, dass Verdrehungen des Pfannenimplantats innerhalb der künstlichen Gelenkpfanne ausgeschlossen sind und es daher nicht zu Abrieb am Knorpelgewebe kommen kann.

Die Erfindung wird nachfolgend anhand von Ausführungsbeispielen und der beiliegenden, sich auf diese Ausführungsbeispiele beziehenden Zeichnungen näher erläutert. Es zeigen:
- Fig. 1: ein erfindungsgemäßes Pfannenimplantat in einer Seitenansicht,
- Fig. 2: das Pfannenimplantat von Fig. 1 in einer Draufsicht,
- Fig. 3: ein weiteres Ausführungsbeispiel für ein Pfannenimplantat nach der Erfindung in einer Seitenansicht,
- Fig. 4 und 5: ein drittes Ausführungsbeispiel für ein Pfannenimplantat in einer Seitenansicht und einer Draufsicht und
- Fig. 6 und 7: weitere Ausführungsbeispiele für Pfannenimplantate nach der Erfindung in einer Teilansicht von der Seite.

Ein schalenartiges Pfannenimplantat weist eine konvexe Außenfläche 1 und eine konkave Innenfläche 2 auf. Beide Flächen bilden einen Abschnitt einer Kugeloberfläche.

Die Außenfläche 1 ist zur Anlage gegen erhalten gebliebenes Knorpelgewebe einer Hüftgelenkpfanne vorgesehen. Auf der Innenfläche 2 gleitet die konvexe Kugelfläche einer den natürlichen Femurkopf ganz oder teilweise ersetzenden Femurprothese (nicht gezeigt). Die Tiefe des Pfannenimplantats ist so bemessen, dass sein Rand 3 etwa bündig mit dem Rand der natürlichen Hüftgelenkpfanne abschließt.

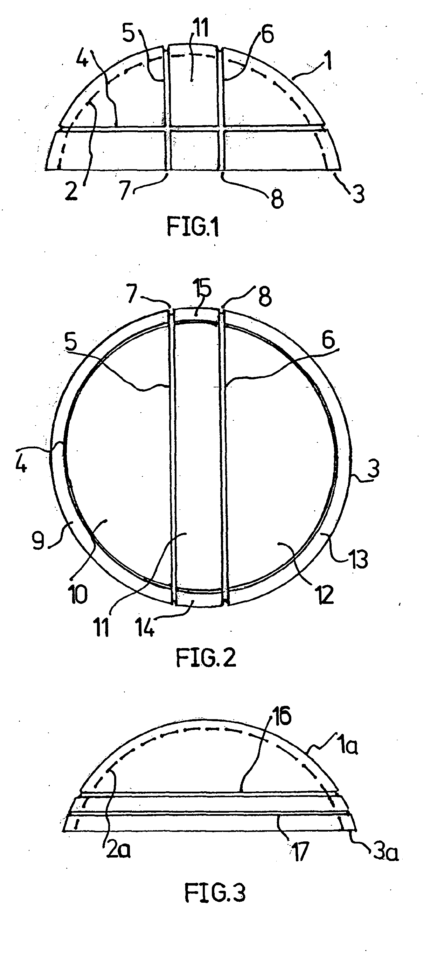

Wie die Fig. 1 und 2 erkennen lassen, sind in der Außenfläche 1 Vertiefungen in Form von Rillen 4 bis 6 gebildet, von denen die Rille 4 als geschlossener Rillenring parallel zum Rand 3 verläuft. Die zueinander parallelen Rillen 5 und 6 schneiden die Rille 4 senkrecht und reichen mit ihren beiden Enden unter Bildung jeweils einer Randöffnung 7 bzw. 8 bis an den Rand 3 heran.

Die an die Rillen 4 bis 6 angrenzenden Kugelabschnittsflächen 9 bis 15 sind in dem betreffenden Ausführungsbeispiel poliert, d.h. der außerhalb der Flächenbereiche der Rillen liegende Teil der Außenfläche 1 ist geglättet.

Nach einer Implantation der in Fig. 1 und 2 gezeigten Pfannenprothese kann der polierte Teil der Außenfläche 1 auf dem Knorpelgewebe in der natürlichen Hüftgelenkpfanne gleiten, d.h. der größte Teil der Knorpelgewebefläche bleibt von Scherbelastungen unbeeinträchtigt. Lediglich im Bereich der Rillen kommt es zu einer solchen, wobei das Knorpelgewebe in die Rillen eindringt und die Pfannenprothese vorteilhaft vor Drehung in der und Austritt aus der natürlichen Hüftgelenkpfanne bewahrt.

In den folgenden Ausführungsbeispielen sind gleiche oder gleichwirkende Teile mit derselben Bezugszahl wie in den Fig. 1 und 2 bezeichnet, wobei der betreffenden Bezugszahl der Buchstabe a, b usw. beigefügt ist.

Ein in Fig. 3 gezeigtes Pfannenimplantat mit einer Außenfläche 1 a, einer Innenfläche 2a und einem Rand 3a weist zwei Vertiefungen in Form geschlossen umlaufender Rillen 16 und 17 auf, welche zueinander und jeweils zu dem Rand 3a des Pfannenimplantats parallel verlaufen.

Während der Querschnitt der Rillen 9 bis 15 der vorangehend beschriebenen Pfannenprothese im Querschnitt etwa rechteckig sind, weisen die Rillen 16 und 17 einen dreieckförmigen Querschnitt auf.

In die Rillen 16 und 17 eindringendes Knorpelgewebe verhindert wie bei dem vorangehenden Ausführungsbeispiel eine Drehung des Pfannenimplantats innerhalb der natürlichen Gelenkpfanne und sichert, dass das Pfannenimplantat in der Gelenkpfanne verbleibt. Die an die Rillen 16 und 17 angrenzenden Kugelflächenbereiche der Außenfläche 1 a sind wie bei dem vorangehenden Ausführungsbeispiel poliert.

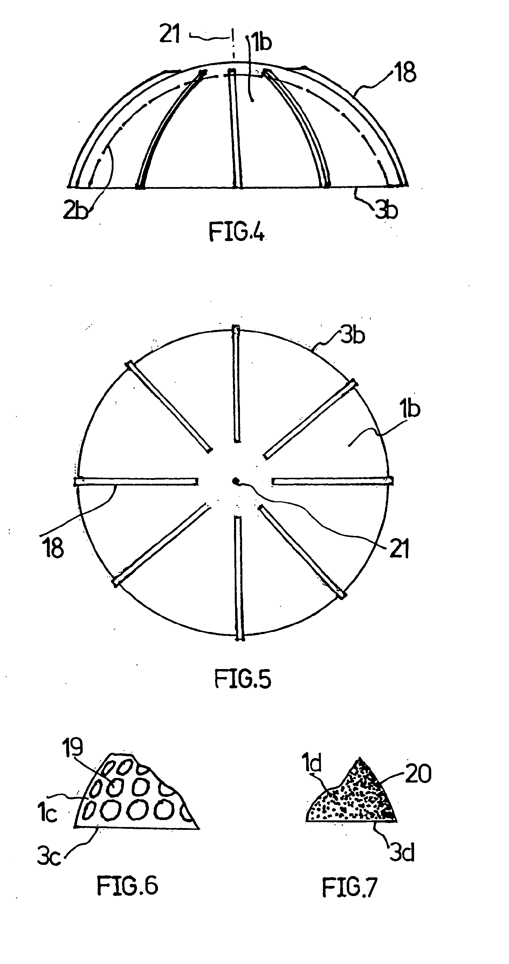

Bei dem Ausführungsbeispiel von Fig. 4 und 5 sind auf einer Außenfläche 1 b eines Pfannenimplantats anstelle von Vertiefungen insgesamt acht Vorsprünge in Form von Stegen 18 gebildet, welche sich in der in Fig. 5 gezeigten Draufsicht radial von einer Rotationssymmetrieachse 21 des schalenförmigen Pfannenimplantats zu dessen Rand 3b hin erstrecken. Während ein Ende der Stege 18 jeweils bis an den Rand 3b heranreicht, ist das andere Ende jeweils vom Polpunkt der Außenfläche 1 b entfernt.

Beim Einsetzen des in den Fig. 4 und 5 gezeigten Implantats in die natürliche Hüftgelenkpfanne dringen die Stege 18 in das in der Hüftgelenkpfanne verbliebende Knorpelgewebe ein. Die radiale Lage der Stege 18 behindert das Einsetzen des Pfannenimplantats in Richtung der Rotationssymmetrieachse 21 nicht. Die in das Knorpelgewebe eingedrungenen Stege 18 verhindern sowohl eine Drehung um diese Achse als auch um den Kugelmittelpunkt der Außenfläche 1 b, die zu einem Austritt des Implantats aus der Hüftgelenkpfanne führen würde.

Ein in Fig. 6 gezeigtes Pfannenimplantat mit einem Rand 3c weist auf seiner Außenfläche 1 c Vertiefungen 19 entsprechend der Oberflächenstruktur eines Golfballs auf. Anstelle der Vertiefungen könnten auch noppenartige Vorsprünge gebildet sein.

Der die Vertiefungen 19 umgebende übrige Teil der Außenfläche 1 c ist poliert.

Ein in Fig. 7 gezeigtes Pfannenimplantat mit einer Außenfläche 1 d und einem Rand 3d weist eine durchgehende Aufrauung 20 auf, welche z.B. durch ein Netz eng beieinander liegender Rillen ähnlich einer gerändelten Fläche gebildet sein kann.

Es versteht sich, dass auf einer Außenfläche eines Pfannenimplantats sowohl Vertiefungen als auch Vorsprünge gebildet sein können.

Ein weiteres Ausführungsbeispiel könnte lediglich am Polpunkt der Außenfläche eine Noppe aufweisen, die in das Knorpelgewebe der natürlichen Hüftgelenkpfanne eindringt, jedoch, wie auch Steg 18 bei dem Ausführungsbeispiel von Fig. 4 und 5, die Knorpelgewebeschicht nicht durchdringt.

## Patentansprüche

1. Implantat zur Bildung einer künstlichen Hüftgelenkpfanne, mit einer konvexen Außenfläche (1), die zur Anlage gegen in der natürlichen Hüftgelenkpfanne verbliebenes Knorpelgewebe vorgesehen ist,
**dadurch gekennzeichnet,**
**dass** die Außenfläche (1) wenigstens eine Knorpelgewebe aufnehmende Vertiefung (4-6;16,17;19) oder/und wenigstens einen in das Knorpelgewebe eindringenden Vorsprung (18) aufweist.

2. Implantat nach Anspruch 1,
**dadurch gekennzeichnet,**
**dass** der an die Vertiefung (4-6;16,17;19) oder/und den Vorsprung angrenzende übrige Teil (9-15) der Außenfläche (1) geglättet ist.

3. Implantat nach Anspruch 2,
**dadurch gekennzeichnet,**
**dass** der geglättete Teil (9-15) der Außenfläche (1) größer als der von der Vertiefung (4-6;16,17;19) oder/und dem Vorsprung eingenommene Teil der Außenfläche (1) ist.

4. Implantat nach einem der Ansprüche 1 bis 3,
**dadurch gekennzeichnet,**
**dass** die Vertiefung länglich, vorzugsweise als Rille (4-6;16,17), und dass der Vorsprung länglich, vorzugsweise als Steg (18), ausgebildet ist.

5. Implantat nach einem der Ansprüche 1 bis 4,
**dadurch gekennzeichnet,**
**dass** die Vertiefung (4;16,17) einen unterbrochenen oder geschlossenen Ring bildet.

6. Implantat nach Anspruch 5,
**dadurch gekennzeichnet,**
**dass** der Ring parallel zum Rand (3,3a) der Außenfläche (1,1a) verläuft.

7. Implantat nach einem der Ansprüche 1 bis 6,
**dadurch gekennzeichnet,**
**dass** der Vorsprung (18) in Projektion auf eine zu einer Rotationssymmetrieachse (21) des Implantats senkrechte Produktionsfläche radial zur Rotationssymmetrieachse (21) verläuft.

8. Implantat nach Anspruch 7,
**dadurch gekennzeichnet,**
**dass** sich der Vorsprung (18) durchgehend oder mit wenigstens einer Unterbrechung von einem Punkt des Randes (3b) der Außenfläche (1 b) bis zu einem dem Punkt diametral gegenüberliegenden Punkt des Randes (3b) der Außenfläche (1b) erstreckt.

9. Implantat nach einem der Ansprüche 1 bis 8,
**dadurch gekennzeichnet,**
**dass** auf der Außenfläche (1) sich kreuzende Rillen (4;5,6) oder/und Stege gebildet sind.

10. Implantat nach einem der Ansprüche 1 bis 9,
**dadurch gekennzeichnet,**
**dass** die Außenfläche (1d) eine, vorzugsweise die gesamte Außenfläche (1d) durchgehende Aufrauhung (20) aufweist.
